# EUROPEAN PATENT APPLICATION

(11) **EP 2 023 142 A1**
(43) Date of publication of application: **11.02.2009**
(21) Application number: 07014871.3
(22) Date of filing: 30.07.2007
(51) Int. Cl.: G01N 33/569

(54) **Immunoassay utilizing recombinant nucleocapsid-proteins for detection of antibodies to human coronaviruses**

(71) Applicant: Mikrogen Molekularbiologische Entwicklungs-GmbH, 82061 Neuried (DE)
(72) Inventor: Motz, Manfred, 80689 München (DE)
(74) Representative: Keller, Günter

(57) **Abstract**

A test kit for the immunological diagnosis of a SARS virus infection in a sample is disclosed which comprises at least a fragment of the nucleocapsid protein N of a SARS virus and at least one fragment of a nucleocapsid protein N of another coronavirus selected from the group comprising the coronaviruses NL63, 229E, HKU1 and OC43.

## Description

Coronaviruses (family Coronaviridae, order Nidovirales) are large (120 to 160 nm), enveloped, roughly spherical particles. They possess a non-segmented, single-stranded RNA genome of up to 31 kb in positive orientation. Based on serological cross-reactivities and sequence analysis, coronaviruses are classified in three distinct groups. Coronaviruses are known to infect many domestic animals as well as humans, causing acute and chronic diseases of the respiratory, enteric and central nervous system. Human coronaviruses (HCoVs) 229E and OC43 have first been described in the 1960s and are thought to be responsible for 10 to 30% of all common colds.

The new infectious disease SARS (Severe Acute Respiratory Syndrome) emerged in November 2002 in Guangdong Province, People's Republic of China. Within a few months, SARS had spread to 26 countries, infecting more than 8,000 people of whom almost 10% died. The etiological agent was recognized to be a novel virus, termed SARS-CoV [Drosten et al. (2003) New Engl. J. Med., 1967-1976], which is related to, but distinct from other known coronaviruses. Recent findings suggest a close relationship between SARS-CoV and group II-coronaviruses [Kim et al., J. Microbiol. (2006)], pp. 83-91.

In 2004, van der Hoek et al. [Nat. Med. (2004), 368-373] reported the isolation of a new group I-CoV from a seven-month-old child with bronchiolitis, named HCoV-NL63. Independently described viruses HCoV-NL [Fouchier et al., (2004), PNAS, 6212-6216] and HCoV-NH [Esper et al., (2005), J. Infect. Dis., 492-498, are closely related to NL63 and are likely to represent strains of the same species of virus.

HCoV-NL63 has been linked to more severe ailments, such as morbus croup and a systemic vasculitis in young children, Kawasaki disease, although association with the latter is discussed controversially. NL63 infections have been reported throughout the world indicating that this HCoV can be regarded as a new major etiologic agent of respiratory disorders. HCoV-HKU1 was first isolated 2005 in Hongkong from a 71-year-old man suffering from pneumonia and was classified as group II-coronavirus [Woo et al., (2005), J. Virol. pp. 884-895]. So far, this strain has been detected in Asia, Europe, USA and Australia in connection with respiratory and enteric tract illness.

WO 2004/085633 discloses a novel human virus causing severe acute respiratory syndrome (SARS) and uses thereof. The complete genomic sequence of the hSARS virus is disclosed in this international application. Furthermore, nucleic acids derived from the hSARS virus and their use in diagnostic methods is disclosed.

WO 2005/016132 discloses diagnostics for SARS virus whereby recombinant proteins, in particular the nucleocapsid (N) protein and spike (S) protein as well as fragments thereof were used.

Much of diagnostics relating to coronaviruses was and still is based on nucleic acid technologies (NATs). As it appears that reconvalescence leads to a loss of viremia, NATs are not applicable in screening for coronavirus epidemiology outside of acute pandemics (e.g. SARS). Serology so far is rarely used and not yet developed to distinguish between pathogenic types of human coronaviruses.

It is an object of the present invention to provide a method of diagnosing whether a patient suffers from a virus belonging to the SARS group or whether the virus belongs to another coronavirus. Further suitable testkits are provided.

As candidate antigens for immunoassays detecting coronaviral antibodies, structural proteins are of special interest. The RNA binding nucleocapsid (N) protein shows high immunogenic activity and is abundantly produced during infection. Generally the N-protein binds to the RNA genome of the virus. The N-Protein has a size of about 50 to 60 KD. Several methods using nucleocapsids as diagnostic antigens have been designed with special emphasis on SARS-N. Here, an immunoassay, preferably a line-immunoassay, for rapid and simultaneous detection of antibodies directed against the five presently known HCoVs (229E, NL63, OC43, HKU1, SARS) in human sera based on recombinant viral N proteins is disclosed.

For this purpose, the corresponding viral genes were cloned and expressed in *Escherichia coli*. Purified proteins were subsequently employed in the line-immunoassay and tested for suitability as diagnostic antigens.

The present invention provides in one embodiment a test kit for the immunological diagnosis of a SARS virus infection in a sample which comprises at least a fragment of the nucleocapsid protein N of a SARS virus and at least one fragment of a nucleocapsid protein N of another coronavirus selected from the group comprising the coronaviruses NL63, 229E, HKU1 and OC43.

Several variants of the SARS virus have been isolated and identified. In the test kits of the present invention a suitable fragment of a nucleocapsid protein N derived from a SARS virus may be used. One typical representative of a SARS virus has been described in WO 2004/085633. The virus has been deposited with the China Centre for Type Culture Collection (CCTCC) under the accession number CCTCC-V200303 on April 2, 2003. The sequence of the SARS virus is disclosed in WO 2004/085633.

The nucleic acid sequence of a 1269 bp of nucleocapsid (N) was deposited with NCBI Entrez accession number AY283798. This sequence was derived from SARS strain SIN2774. The nucleotide sequence of SIN2774 (accession number AY283798) is accessible via e.g. http://www.ncbi.nim.nih. gov/entrez/query.fcgi (Nucleotide); [Ryan, Y, Wei, C.L. et al., *Comparative whole genome sequence analysis of 9 SARS coronavirus isolates show mutations in functional domains and potential geographical variations.*

In the test kit of the present invention the nucleocapsid protein N is used either as complete protein having about 423 amino acids or in the form of fragments of this protein. The fragment to be used in the test kit has a length of at least 50 amino acids, preferably at least 100 amino acids, more preferably at least 200 amino acids and most preferred at least 400 amino acids. In an especially preferred embodiment the complete nucleocapsid N is used.

The nucleocapsid protein N may be derived from any SARS virus which is already known or which is classified to belong to the species of SARS virus. The homology of a nucleocapsid protein N of a SARS virus which can be used in the course of the present invention has a homology of at least 90%, preferably at least 95% identity calculated over the whole length of the complete nucleocapsid protein N to the reference sequence of SIN2774.

Furthermore, the test kit of the present invention comprises at least one fragment of a nucleocapsid protein N of another coronavirus which is selected from the group comprising the coronaviruses NL63, 229E, HKU1 and OC43 and variants thereof.

The nucleocapsid protein N of the coronaviruses to be used in the test kit do have a length of at least 50 amino acids, preferably at least 100 amino acids, more preferably at least 200 amino acids and most preferred at least 400 amino acids. In an especially preferred embodiment the complete nucleocapsid protein N of the other coronaviruses is used.

In an especially preferred embodiment the full-length nucleocapsid protein N is used together with at least one partial fragment of a nucleocapsid protein N derived from another coronavirus. Said fragment(s) has/have a length of at least about 100 amino acids.

The coronaviruses NL63, 229E, HKU1 and OC43 are already known and the sequences are available from data banks. The test kit of the present invention comprises at least one nucleocapsid protein N derived from one coronavirus selected from the above-mentioned group. Preferred is, however, the use of two nucleocapsid proteins N obtained from two different coronaviruses, more preferred from three different coronaviruses and most preferred from four different coronaviruses whereby the group of coronaviruses preferably consists of the viruses NL63, 229E, HKU1 and OC43. The nucleotide sequence of protein N of HKU1 strain Caen is available under GenBank accession number DQ778921.

The present invention relates to immunological assays whereby the nucleocapsid protein N is linked to a solid carrier phase which consists preferably of nitrocellulose, nylon, polystyrol and other materials usually used in immunological tests.

In an especially preferred embodiment of the present invention the test kit is present in the form of a line-assay which means that the different proteins are deposited as well-defined bands on solid support, preferably a nitrocellulose stripe.

The present invention relates also to a method for detecting antibodies which are specific for a SARS virus in a sample. Usually the sample is obtained from a patient and may be blood, serum, saliva, sputum or other liquids or solid substances obtained from a patient. The method is performed in vitro outside the body of the patient. In the method the sample is brought into contact with a test kit as disclosed in the present invention. With the help of this method it can be easily decided whether the patient is infected by a SARS virus or a rather harmless coronavirus. In a preferred embodiment of the present invention the method is performed with the test kits described herein.

The SARS epidemic of 2002 and 2003 led to a boost in coronavirus research. Two so far unknown viruses, NL63 and HKU1, have since been identified and detection of further HCoVs is likely. Most diagnostic assays use RT-PCR techniques, while test systems based on serology distinguishing between all types of HCoVs are, to our knowledge, almost completely missing. Yet, studies regarding coronavirus epidemiology are of great interest. However, techniques such as nucleic acid diagnostics are not suitable, since coronaviral RNA can only be detected during the acute phase of infection or shortly thereafter. When clinical symptoms are mild or missing altogether, serology opens the possibility to trace down silent converters, which in the case of SARS is very valuable information on the actual virus distribution among the population.

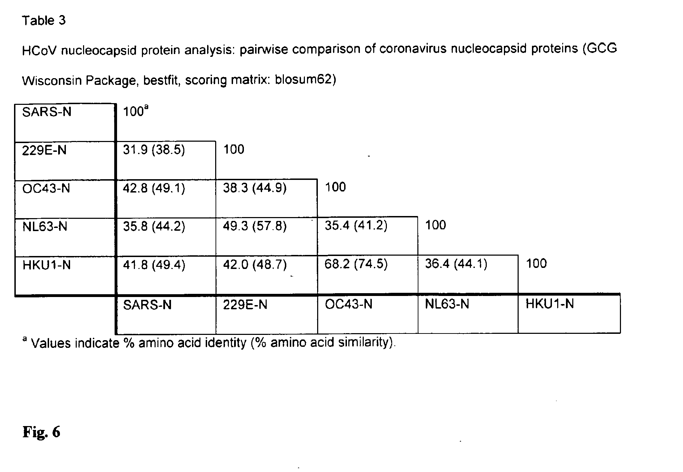

Sequence comparisons revealed relatively low sequence identities and similarities as shown in Table 3 (Fig. 6). Nucleocapsid proteins were selected as appropriate antigenic markers for retrospective diagnosis of coronaviral infections in humans. The highest amino acid sequence identities are present between HKU1- and OC43-encoded nucleocapsids (68.2% identity, 74.5% similarity). N protein sequences of all other coronaviruses considered as antigens in our immunoassay, exhibit much lower amino acid identities when compared to each other (31.9% to 49.3% identity; 38.5% to 57.8% similarity;Table 3).

Therefore, an immunoassay, preferably a line-immunoassay using recombinant N proteins as antigens to specifically detect coronavirus-directed antibodies in human sera was developed.

Validation of the newly developed test was carried out by assaying well-defined sera of patients who suffered from various coronaviral infections. Consistently, all available human sera sampled from convalescent patients (tested positive for HCoVs 229E, OC43 or SARS-CoV by microneutralization experiments, western blotting, ELISA or IFA) reacted with their respective antigens as expected, indicating an excellent sensitivity of the preferred line-immunoassay.

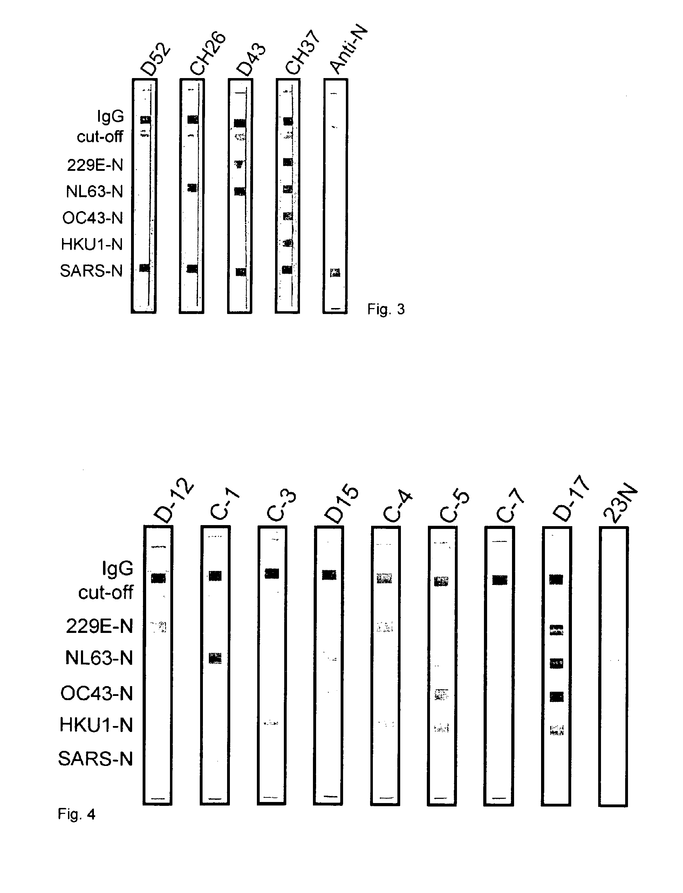

Although antigenic cross-reactivity to SARS nucleocapsid has been described earlier, any serological cross-reactivity with SARS-N could not be detected, even in those cases where donors strongly responded to the other four nucleocapsids (Fig. 4, D-17).

Accordingly, only sera obtained from convalescent SARS patients reacted with the SARS antigen. Moreover, antibody binding reactions to SARS-N were consistently strong, as demonstrated in Fig. 3. In the line-immunoassays one single distinct band is shown proving presence of anti-SARS-CoV antibodies (Fig. 3, e.g. D52). There is therefore no evidence of unspecific binding to antigens of other HCoVs in convalescent SARS sera. Thus, SARS N protein provides a powerful tool to differentiate between SARS infection and infections with other HCoVs in serological studies.

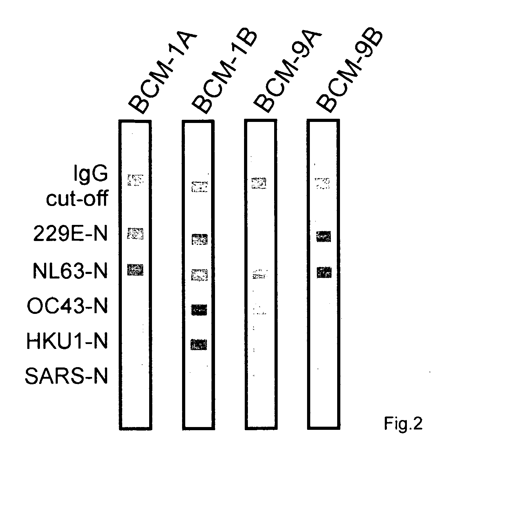

However, cross-reactions among other HCoVs might occur. N proteins of OC43 and HKU1 (group I HCoVs) display the highest amino acid identity among all coronaviruses (68.2%, Table 3) and non-specific binding of antibodies directed against OC43-N to HKU1-N is possibly observed (Fig. 2, BCM-1A/B, Table 2). Donors of these serum samples have not been tested for HKU1 contagion (samples were characterized before primary detection of HKU1). However, double infection with OC43 and HKU1 seems unlikely, since simultaneous detection of HKU1- and OC43-directed antibodies occurred in all but one serum pair (BCM-3A/B).

Nonetheless, evidence was found of IgGs recognizing OC43-N, without binding to HKU1-N (Fig. 4, D-15), contradicting a general cross-reactivity. Non-specific cross-reactions between 229E and NL63 (group I HCoVs) antigens appear rather unlikely since amino acid identity between both nucleocapsids is quite low (49.3%, Table 3). Nevertheless, in a single 229E-positive serum sample, NL63 antibody reaction seemed to be co-induced (BCM-9A/B: Table 2, Fig. 2). The other defined sera sampled from 229E-infected patients (BCM-7 to BCM-12) consistently indicated antibody binding signals to NL63-N. These sera were initially not characterized for presence of NL63 viral agent, since NL63 was actually not discovered at sampling time. Thus, proof of NL63-specific antibodies in these sera is not necessarily evidence for cross-reactivity. Co-infections of 229E and NL63, or previous contact with NL63 earlier in the lives of these patients, resulting in basal antibody levels, cannot be excluded.

Furthermore, positive reactions with OC43-N and HKU1-N in sample BCM-10B (Fig. 2, Table 2) could be due to a true HCoV-OC43 infection.

Antibodies specific for human coronaviruses 229E and OC43 can be found in about 90% of adults throughout the world. While immunological data concerning seroprevalence of anti-HCoV-229E and anti-HCoV-OC43 antibodies are available, information about humoral responses concerning recently discovered HCoV-NL63 and HCoV-HKU1 is rather scarce, despite their widespread association with respiratory tract infections.

The global SARS epidemic of 2002 to 2003 was controlled by a highly successful public health effort and there were no more reported cases since April 2004. Thus, it appears that SARS-CoV is currently not circulating in humans. However, studies on the actual distribution of SARS-CoV are lacking and surveys screening healthy donors for antibodies specific for SARS-CoV are rare. Contact leading to seroconversion may result in asymptomatic or subclinical (nonpneumotic) manifestations and are therefore not recognized as SARS infections. Hence, serology remains an important tool in SARS diagnosis.

By testing 25 arbitrarily selected sera, it was assumed that most samples collected from adults would exhibit positive IgG reactions to at least one, but possibly several, coronavirus nucleocapsids, since sustained coronavirus infections become more likely with increasing age. Consistent with these assumptions, sera were found recognizing all antigens (except SARS) as well as samples showing evidence of infection with only one type of virus. Furthermore, three negative sera were identified (2 children, 1 adult). The children most likely never had contact to coronaviruses, while the seronegative adult might have had coronavirus infections earlier in his life, which became non-detectable as anti-nucleocapsid IgG titres decrease over time.

The line-immunoassay approach is considered to be highly favourable for large-scale epidemiological studies on coronaviruses. The assay system allows rapid detection of antibodies against all human coronaviruses known so far in a single experiment with one antigen-coated strip, requiring only very low serum volumes (15 µl).

Assay set up and test strip production is straightforward and cost effective. N proteins are appropriate antigens, since they are extremely immunogenic and provide a robust tool for antibody detection with adequate sensitivity. Yet, the data also indicate that cross-reactive epitopes among HCoVs within group I or group II might be involved in IgG response to HCoV nucleocapsids. To gain even better specificity and to exclude possible false positive results, the antigen portfolio on the strips could be extended.

### Figure Legends

Figure 1 shows Table 1 wherein the accession number of the genes used in the present invention are given together with suitable primers and cloning sites.
Figure 2: Evaluation of the line-immunoassay with clinically defined serum pairs from HCoV-OC43 (BCM-1A/B) or HCoV-229E (BCM-9A/B)-infected patients. Membrane strips were coated with recombinant N proteins and controls as described in materials and methods. Representative immunoassay strips are shown. BCM-1A: serum from acute phase of HCoV-OC43 infection; BCM-1B: serum from convalescent phase of HCoV-OC43 infection, collected 55 days after BCM-1A. BCM-9A: serum from acute phase of HCoV-229E infection; BCM-9B: serum from convalescent phase of HCoV-229E infection, collected 116 days after BCM-9A. Sera taken during acute phase of HCoV-infection, no OC43- or 229E-specific antibodies could be detected, whereas samples collected after convalescence strongly reacted with the corresponding antigens. Co-reaction with HKU1 nucleocapsid (BCM-1) or NL63 nucleocapsid (BCM-9) might be due to cross-reactions.
Figure 3: Line-immunoassay evaluation probing sera sampled from convalescent SARS patients. A representative selection of immunoassay strips out of 49 samples is shown. Without exception, all samples clearly display strong signals to SARS nucleocapsid as expected. In addition, various combinations of reactions with other HCoV-strains are shown. Anti-N: serum from rabbits immunized with recombinant SARS-CoV nucleocapsid was used in the assay. The immunoassay demonstrates a distinct and specific reaction with SARS N.
Figure 4: Arbitrarily selected sera from healthy donors were applied in the line-immunoassay. Single, double and multiple anti-coronaviral immune responses in various combinations could be verified as shown. In addition, blood samples without seroconversion could be identified (C-7). 23N: serum from rabbits immunized with truncated HCoV-NL63 nucleocapsid was used.
Figure 5: Table 2 shows reactions of clinically defined sera with HCoV nucleocapsids.
Figure 6: Table 3 shows a HCoV nucleocapsid protein analysis.

### Example 1: Cloning and expressing of N-proteins

### 1.1 Viral strains and viral RNA

Human coronaviruses 229E (ATCC VR-740) and OC43 (ATCC VR-759) were obtained from the American Type Culture Collection (Manassas, VA, USA) and propagated on MRC-5 cells. RNA was isolated from cell culture supernatant using the SV Total RNA Isolation System (Promega, Mannheim, Germany). HCoV-NL63-RNA was kindly provided by L. van der Hoek, Amsterdam, Netherlands. HKU1-RNA (strain Caen) was received from A. Vabret, Caen, France and SARS-CoV-RNA (strain FRA) was prepared and sent to our laboratory by M. Eickmann, Marburg, Germany.

### 1.2 RT-PCR, PCR and cloning of coronaviral nucleocapsid genes

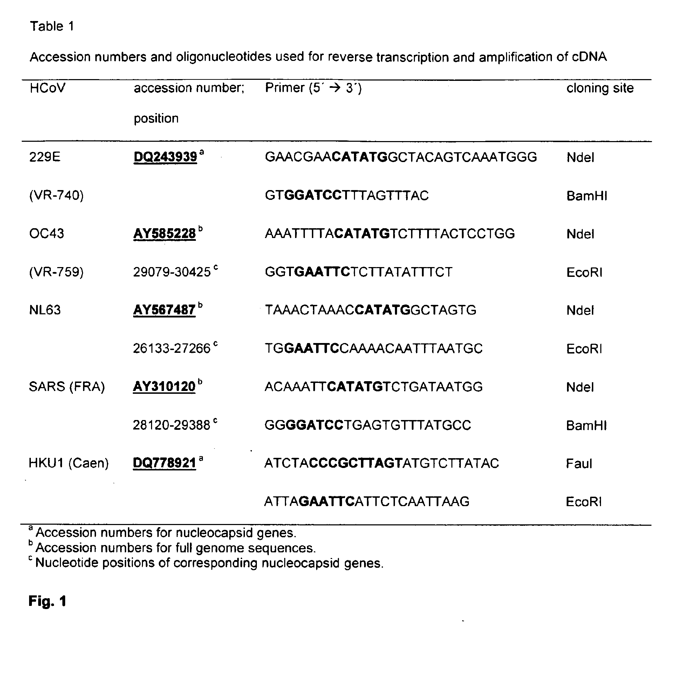

cDNA was synthesized by M-MLV reverse transcriptase, (Promega) using gene specific reverse primers derived from full genome sequences (see Fig. 1 (Table 1)) for GenBank accession numbers). Subsequently, cDNA was amplified utilizing PfuTurbo®hotstart DNA polymerase (Stratagene, Heidelberg, Germany) with primer pairs displayed in Table 1. Cloning sites overlapping the start codon and cloning sites downstream of the stop codon were introduced as indicated. All oligonucleotides were synthesized by Metabion, Martinsried, Germany.

The nucleotide sequence of the N gene of HCoV-HKU1 strain Caen was deposited in the GenBank sequence database under accession number **DQ778921.** GenBank accession numbers are listed in Table 1 (Fig. 1).

The resulting PCR-products were cloned into the expression vector pCS04 which is a derivative of pET22b (Novagen, Schwalbach, Germany), encoding six N-terminal histidine residues for purification. Integrity of reading frames of generated expression constructs pCS04/229E-N, pCS04/OC43-N, pCS04/NL63-N, pCS04/HKU1-N and pCS04/SARS-N were confirmed by DNA sequencing (Geneart, Regensburg, Germany). Chemically competent *Escherichia coli* BL21-CodonPlus®(DE3)-RP (Stratagene) were transformed with the constructed expression plasmids.

### 1.3 Sequence analysis of cloned genes

Cloned nucleotide sequences of 229E-N, OC43-N, NL63-N, and SARS-N matched completely with published sequences, whereas the nucleocapsid gene of coronavirus HKU1 differed slightly from already published HKU1 sequences. For RT-PCR amplification of the HKU1 N gene, viral RNA isolated from a nasal aspirate, sampled from an infant with respiratory infection in Caen, France (Vabret, A., personal communcation), was used. The newly identified protein sequence (corresponding to GenBank accession number **DQ778921)** exhibits 97.3% amino acid identity and 98.6% amino acid similarity to the N protein of HKU1, strain N23 (accession number **DQ437631).** Genetic variability among HCoV-HKU1 isolates has been studied previously. For further classification of HCoV-HKU1, strain Caen, sequencing of the S and pol genes will be necessary.

### Example 2

### Expression of N genes and purification of recombinant N proteins

Transformed cells were grown in Luria-Bertani medium (supplemented with 100 µg/ml ampicillin and 34 µg/ml chloramphenicol) to an absorption at 600 nm of ∼0.6 at 37°C under vigorous shaking. Nucleocapsid protein synthesis was induced by addition of isopropylthio-β-D-galactoside (final concentration 1 mM). After 3 h, cells were harvested by centrifugation (10800 x g, 10 minutes, 4°C) and stored at -80°C.

Nucleocapsid proteins of 229E, NL63 and SARS were expressed in highly soluble form, whereas OC43-N and HKU1-N had to be purified from inclusion bodies. Therefore, all buffers used for purification of OC43-N and HKU1-N were supplemented with 1 M or 3 M urea, respectively. All buffers contained 50 mM sodium phosphate, pH 8.0. Frozen cells (6 to 8 g wet weight) were thawed and suspended in loading buffer containing 500 mM NaCl and 20 mM imidazol at 10 ml per gram wet weight. To inhibit protease activity, Pefabloc SC® (Biomol, Hamburg, Germany) was added before disruption of cells to a final concentration of 2 mM. Cells were lysed by two passages through the high pressure system Basic-Z (IUL Instruments, Königswinter, Germany) at 1500 bar. To avoid DNA contamination, the crude extract was first incubated with 2 U/ml Benzonase® (Merck, Darmstadt, Germany) for 20 minutes and then precipitated with 0.7% (w/v) protamin sulphate (Sigma-Aldrich, Steinheim, Germany). The lysate was cleared by centrifugation (45000 x g, 10 minutes, 4°C). Soluble, histidine-tagged nucleocapsid proteins (229E-, NL63- and SARS-N), present in the supernatant, were then purified by affinity chromatography using a Ni-Sepharose column (bed volume 17 ml; GE Healthcare, Munich, Germany). Bound proteins were eluted with a linear gradient of imidazol (20 mM to 500 mM) in loading buffer (500 mM NaCl). The nucleocapsid containing fractions (350-450 mM imidazol) were pooled, dialysed (50 mM NaCl) and applied on a SP-Poros ion-exchange column (bed volume 1.6 ml; Perseptive Biosystems, Framingham, MA, USA). Elution was conducted with a linear gradient from 50 mM NaCl to 1 M NaCl. Nucleocapsid fractions (500-900 mM NaCl) were pooled again and loaded on a preparative Superdex 75 gelfitration column (bed volume 120 ml; 16/60 Superdex^{™} 75 prep grade, GE Healthcare), equilibrated in loading buffer (150 mM NaCl). The nucleocapsid containing fractions were pooled, aliquotted and stored frozen at -80°C.
Insoluble proteins OC43- and HKU1-N were treated analogously under denaturing conditions as described above. High protein purity was confirmed by analysis on a coomassie-stained polyacrylamid gel. Nucleocapsid protein concentration was determined by the BCA method (Sigma-Aldrich) or spectro-photometrically.

### Instrumentation

Affinity chromatography and gelfiltration were performed by use of the High Load^{™}-System (Pharmacia, Freiburg, Germany). Ion-exchange-chromatography was run on a BioCad 700E workstation (Perseptive Biosystems).

Nucleocapsid genes of all five known human coronaviruses were heterologously expressed in *Escherichia coli* and purified to homogeneity. Highly purified recombinant antigens were obtained. Lack of any interfering protein contamination originating from the expression host was demonstrated by western blotting. In purified nucleocapsid fractions, bacterial proteins were not detected by mouse anti*-Escherichia coli* antibodies, while recombinant proteins could be traced during the purification process by use of antibodies recognizing the N-terminal histidine tag (data not shown).

### Example 3

### Production of immunoassay strips

Nucleocapsid antigen solutions (NL63-, OC43- and SARS-N: 50 ng/µl; 229E-N: 25 ng/µl; HKU1-N: 15 ng/µl) and appropriate controls (strongly positive control: human IgG 100 ng/µl; weakly positive control: human IgG 3.5 ng/µl) were immobilized as parallel lines on nitrocellulose transfer membranes (Protran 0.45 µm; Schleicher & Schuell, Dassel, Germany) using the Accutran ACC100/0 cross-blot system (Schleicher & Schuell). Recombinant antigens were incubated on the membrane for 2.5 hours at room temperature under soft shaking. Non-specific binding sites were blocked with 0.2% I-BIock^{™} (w/v) (Tropix, Bedford, USA) supplemented with 1% (v/v) Tween 20 for 2 hours at 37°C. Finally, membranes were air-dried, cut in strips (3 mm x 57 mm) and stored frozen (-20°C) until used.

### Line-immunoassay development and evaluation

Recombinant proteins were immobilized as horizontal lines on membrane strips. Each antigen was tested individually for the optimum concentration to be applied. Best signal intensities and background to signal ratios were achieved by using protein concentrations of 15 to 50 ng/µl. Reliability of the testing system was improved by including a strongly positive (human IgG; 100 ng/µl) and a weakly positive control (human IgG; 3.5 ng/µl) on each strip. The weakly positive control represents a cut-off control. Only signals of the same intensity as or stronger than the defined weakly positive control were considered positive.

Immunoassay performance was tested with three different sample pools: serum pairs of patients who suffered from infections with HCoV-OC43 (BCM-1A/B to BCM-6A/B, n=12) or HCoV-229E (BCM-7A/B to BCM-12A/B, n=12); sera gathered from convalescent SARS patients (n=49); randomly selected specimen from healthy donors (n=25; 18 adults, 7 children). Unfortunately, no human sera collected after confirmed HCoV-NL63 or HCoV-HKU1 infection were available. Additionally, rabbit NL63-N antiserum and rabbit SARS-N antiserum was utilized, showing specific reaction to their respective antigens (Fig. 3, Anti-N and Fig. 4, 23N). All experiments were conducted at least in triplicate.

### Example 4: Evaluation of assay stripes with human sera

Acute and convalescent phase sera from 6 patients with recent HCoV-OC43 infection and from 6 patients with recent HCoV-229E infection were obtained from the serum bank of the Respiratory Pathogen Research Unit of the Baylor College of Medicine, Houston, TX, USA (R.B. Couch). These samples were tested by ELISA as well as microneutralization experiments and convalescent phase sera were shown to have high OC43- and 229E-specific antibody titres. Samples from convalescent SARS-patients were provided by the Chinese Center for Disease Control (China CDC), Beijing, P. R. China. These sera were collected from hospitalized patients during the SARS outbreak of 2003 in Inner Mongolia, P.R. China. SARS-CoV infection was previously confirmed by enzyme-linked immunosorbent assay (ELISA), western blotting and immunofluorescence assay (IFA). Further serum samples used for this study were retrieved from healthy donors, stored at the serum bank of the University of Regensburg, Germany.

### Antibodies and antisera

To screen for contamination and to detect recombinant protein during purification steps, polyclonal rabbit anti-*Escherichia coli* antibodies (Dako, Hamburg, Germany; 1.3 mg/ml) and mouse Penta His^{™} antibodies (Qiagen, Hilden, Germany) were used (dilution 1:1000). We further raised anti-SARS nucleocapsid antibodies by injection of recombinant nucleocapsid protein in rabbits (Davids Biotechnologie, Regensburg, Germany). Experiments were carried out with rabbit SARS-N antiserum at a dilution of 1:10000. Rabbit NL63-N antiserum was provided by L. van der Hoek, Amsterdam, Netherlands and used at a dilution of 1:1000. All primary antibodies and sera were diluted in Tris-buffered saline (TBS), pH 7.5. As secondary antibodies, horseradish peroxidase (HRP)-labelled polyclonal rabbit anti-human-IgG (Dako) was used for detection of anti-N antibodies in human sera. HRP-labelled polyclonal swine anti-rabbit-immunoglobulin (Ig) and rabbit anti-mouse-Ig (Dako; 1.3 mg/ml) were used for detection of corresponding primary antibodies.

### Line-immunoassay arrangement

Prepared immunoassay strips were incubated in human sera (dilution 1:100 in TBS, pH 7.5; total incubation volume: 1.5 ml) for one hour under shaking and washed twice for five minutes in TBS supplemented with 0.05% (v/v) Tween 20 (TTBS). To reduce background and matrix effects, the secondary antibodies were diluted 1:1000 in a buffer containing 70% LowCross^{™}-Buffer (Candor Bioscience, Münster, Germany) and 30% TBS. The strips were then incubated with secondary antibody solution for 45 minutes under shaking. After washing three times for 15 minutes in TTBS, reactions were visualized in NiCl₂/Diaminobenzidine (DAB) solution containing H₂O₂ for three minutes. A dark precipitate indicated a positive response. All steps were performed at room temperature.

### HCoV-OC43 and HCoV-229E

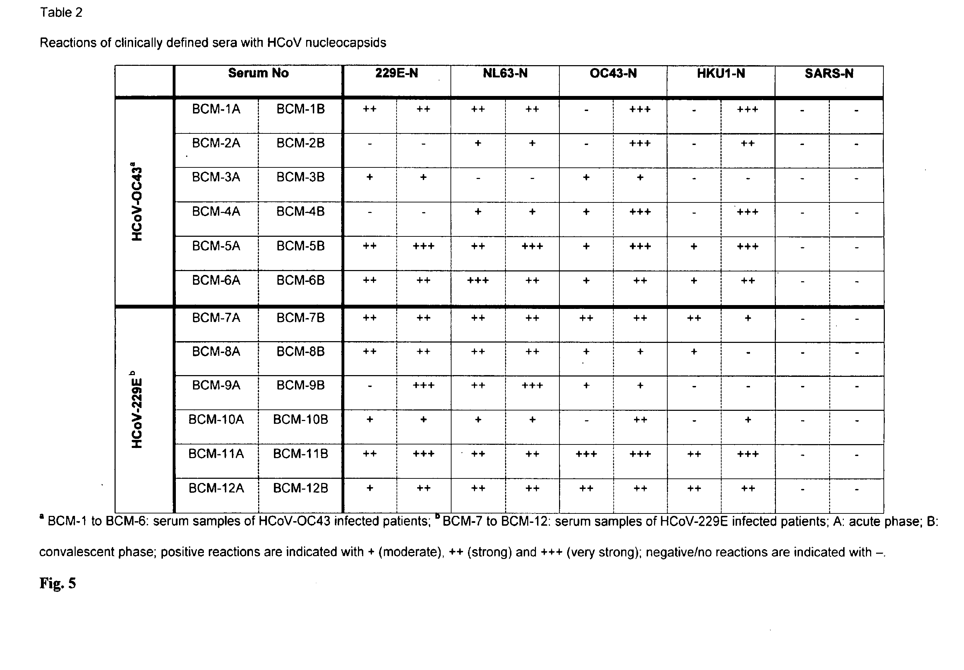

First, HCoV-OC43 and HCoV-229E positive serum pairs were screened regarding two aspects: specific and sensitive reaction to immobilized antigens; comparison of signal intensities (fraction of bound antibodies to relevant antigens) between acute (A) and convalescent (B) phase sera (Fig. 2 and Table 2 (Fig. 5)). All sera obtained from convalescent patients (HCoV-OC43 or HCoV-229E infection) reacted with the respective antigens as expected. A strong induction of humoral responses was observed in 5 out of 6 (OC43) and 3 out of 6 (229E) cases (Table 2). Yet, amplified signal intensities in post-infection OC43 sera (BCM-1 B to BCM-6B) were accompanied by sturdy reactions with the HKU1 antigen, except for BCM-3B, where overall only weak antibody binding signals were detected (Table 2). Reaction intensities of sera derived from HCoV-229E-infected patients increased from acute to convalescent phase in three serum pairs (BCM-9A/B, BCM-11A/B, BCM-12A/B), while in the remaining three sample pairs (BCM-7A/B, BCM-8A/B, BCM-10A/B) no clear boost of antibodies could be detected (Table 2). However, serum BCM-10B, responding only weakly to 229E-N, exhibited distinct reactions to OC43 and to HKU1 N antigens. This observation raises the possibility of a collateral acute infection with HCoV-OC43. In addition, defined sera from HCoV-229E-infected patients (BCM-7 to BCM-12) uniformly showed presence of a basal level of NL63-specific antibodies. Enhancement of antibody response in case of samples BCM-9A/B does not necessarily imply non-specific cross-reactions between 229E and NL63. Remarkably, none of the tested coronavirus positive sera reacted with the SARS antigen (Fig. 2 and Table 2 as shown in Fig. 5).

### SARS-CoV

We further analysed 49 sera attained from convalescent SARS patients. Without exception all samples showed definite strong signals with SARS nucleocapsids. Moreover, 42 (85.7%) specimen responded to HCoV-229E-N, 46 (93.9%) to HCoV-NL63-N, 40 (53.1%) to HCoV-OC43-N and 26 (53.1%) to HCoV-HKU1-N. Representative assay strips indicating the variety of antibody reactions are displayed in Fig. 3.

### Healthy donors

Finally, 25 randomly selected sera from healthy blood donors were tested to investigate natural coronavirus prevalence. Applying these samples in our line-immunoassay, we found evidence of single, double and multiple coronavirus infections in various combinations (Fig. 4). We were also able to identify three blood samples showing no seroconversion, two of which were derived from children (Fig. 4, C-7). In summary, in healthy donors antibody reactions to HCoV nucleocapsids occurred as follows: 229E: 14 (56%), NL63: 15 (60%), OC43: 13 (52%), HKU1: 12 (48%). Again, as expected, no responses to SARS-N could be detected (Fig. 4).

## Claims

1. Test kit for the immunological diagnosis of a SARS virus infection in a sample which comprises at least a fragment of the nucleocapsid protein N of a SARS virus and at least one fragment of a nucleocapsid protein N of another coronavirus selected from the group comprising the coronaviruses NL63, 229E, HKU1 and OC43.

2. Test kit according to claim 1, **characterized in that** the fragment of the nucleocapsid protein N of the SARS virus and/or the coronavirus has a length of at least 50 amino acids.

3. Test kit according to claim 1, **characterized in that** the fragment of the nucleocapsid protein N of the SARS virus and/or the coronavirus has a length of at least 100 amino acids.

4. Test kit according to claim 1, **characterized in that** the fragment of the nucleocapsid protein N of the SARS virus and/or the coronavirus has a length of at least 200 amino acids.

5. Test kit according to claim 1, **characterized in that** the nucleocapsid protein N of the SARS virus and/or the other coronavirus is the complete nucleocapsid protein N.

6. Test kit according to any of the preceding claims, **characterized in that** the test kit comprises at least two fragments of a nucleocapsid protein N of another coronavirus selected from the group comprising the coronaviruses NL63, 229E, HKU1 and OC43.

7. Test kit according to any of the preceding claims, **characterized in that** the test kit comprises at least four fragments of a nucleocapsid protein N of another coronavirus selected from the group comprising the coronaviruses NL63, 229E, HKU1 and OC43.

8. Test kit according to any of the preceding claims, **characterized in that** the test kit comprises the complete nucleocapsid protein N of coronavirus HKU1 and at least one fragment of a nucleocapsid protein N of another coronavirus.

9. Test kit according to any of the preceding claims, **characterized in that** the nucleocapsid proteins N are linked to a solid carrier phase.

10. Test kit according to any of the preceding claims, **characterized in that** it is a line assay.

11. In vitro method for detecting antibodies specific for SARS virus in a sample, **characterized in that** the sample is reacted with a nucleocapsid protein N of SARS and at least one other nucleocapsid protein N derived from another coronavirus selected from the group comprising NL63, 229E, HKU1 and OC43.
